(19) 

Europäisches
Patentamt
European
Patent Office
Office européen
des brevets

(11) **EP 4 059 489 A1**

(12) **EUROPEAN PATENT APPLICATION**

(43) Date of publication:
**21.09.2022   Bulletin 2022/38**

(21) Application number: **21163633.7**

(22) Date of filing: **19.03.2021**

(51) International Patent Classification (IPC):
**A61K 8/34** (2006.01)          **A61K 8/35** (2006.01)
**A61K 8/67** (2006.01)          **A61K 8/45** (2006.01)
**A61Q 19/04** (2006.01)         **A61K 8/37** (2006.01)
**A61K 8/49** (2006.01)

(52) Cooperative Patent Classification (CPC):
**A61Q 19/04; A61K 8/34; A61K 8/342; A61K 8/345;
A61K 8/35; A61K 8/37; A61K 8/45; A61K 8/4993;
A61K 8/678;** A61K 2800/87

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **IONIQ Skincare GmbH & Co. KG
88677 Markdorf (DE)**

(72) Inventors:
 • **LUECK, Annabella
   74906 Bad Rappenau (DE)**
 • **WILLE, Charlotte
   22927 Grosshansdorf (DE)**

(74) Representative: **Lederer & Keller Patentanwälte
Partnerschaft mbB
Unsöldstraße 2
80538 München (DE)**

(54) **ELECTROSTATICALLY SPRAYABLE TANNING FORMULATION**

(57)    The present invention relates to an electrostatically sprayable topical formulation for giving skin a tanned appearance in the absence of ultraviolet radiation exposure or achieving a faster, darker tanned appearance in the presence of ultraviolet radiation exposure that can be easily applied on the skin surface of a subject and shows a sufficient stability, in particular a sufficient storage stability.

Processed by Luminess, 75001 PARIS (FR)

**Description**

The present invention relates to an electrostatically sprayable topical formulation for giving skin a tanned appearance in the absence of ultraviolet radiation exposure or achieving a faster, darker tanned appearance in the presence of ultraviolet radiation exposure that can be easily applied on the skin surface of a subject and shows a sufficient stability, in particular a sufficient storage stability.

Usually a tanned appearance is considered to be a healthy and attractive appearance. Therefore, many persons, especially younger persons, desire to a tanned appearance. Most of them obtain darker skin through exposure to UV light (e.g. sun-tanning or UV lamps). However, it is known that UV exposure results in accelerated skin aging and increase incidence of skin cancer.

Several artificial tanning products are known in the art that offer an alternative to natural or artificial sunlight. Some of them are described for example in US 2 949 403, EP 0 302 147 or EP 2 198 847. The products are also known as sunless tanning products. Sunless tanning products usually contain an active ingredient, such as reducing sugar or colorant, which causes the skin to assume browned appearance.

The sunless tanning products as known in the art come in form of an oil, lotion, or gel, whereby most of the products are lotions. Problems often associated with topical formulations such as tanning formulations include uneven application and streaking effect. If the amount of the tanning formulation, which is applied to the skin surface, is too much or too little, the area may become darker or lighter than the surrounding skin. As a result, the skin coloring may appear unnatural, streaked, or splotchy.

One reason for the uneven application and streaking effect of the tanning formulation is that most of the tanning formulations are applied on the surface of the skin by hand. However, this application method does not ensure that a sufficient amount of the tanning formulation is applied on the skin, in particular this application method does not provide a sufficient coverage of the skin. Moreover, this application method does not avoid that too much of the tanning formulation is applied on the skin surface and thus these areas are darker than the surrounding skin.

Another application method, which should ensure that a sufficient amount of the tanning formulation, but not too much, is applied on the surface of the skin is the use of pump sprays or pre-pressurized aerosol containers so as to have the formulation atomized and sprayed with the aid of propellant gas, as described for example in US 2005/0100516 or US 2003/0094510. However, conventional aerosol sprays frequently employ volatile compounds as propellants, which are environmentally unfriendly, possible hazardous to health and indeed are being legislated against in many countries. Moreover, it is hardly possible to generate a constant and homogenous flow of the tanning formulation. Therefore, also with this application method a uniform and sufficient coverage of the skin surface, i.e. a nearly 100% coverage, is not possible.

In the last few years in the field of cosmetic products a further application method has become of interest. In this application method the cosmetic topical formulation is applied on the subject by electrostatic spraying. Such a method is for example described in WO 94/11119 or WO 2001/012139.

This application method can be carried out without the aid of environmentally unfriendly propellants and provides a constant and uniform flow of the product. Hence, it is possible to apply a sufficient amount of the cosmetic formulation, but not too much, on the surface of the skin and thus a nearly full and uniform coverage of the skin may be possible.

In the prior art different electrostatic spraying devices are known, for example in WO 94/11119, US 2010/0116897 or DE 10 2017 108 610.2 such devices are described. In an electrostatic spraying device a voltage generator creates a high voltage which electrically charges the compounds of the cosmetic product. This results into a spray of the topical cosmetic formulation.

However, most of topical cosmetic formulations like tanning formulations are not electrostatically sprayable, because they do not have appropriate electrical characteristics, e.g. resistivity, permittivity etc., and/or have other properties such like a high surface tension, viscosity etc. which do not permit electrostatic spraying. For example, like many other cosmetic lotions and creams, tanning formulations include emollients, which are poorly electrostatic sprayable.

Emollients, also referred to as cosmetic oils, are compounds that soften and smooth the scales of the skin, which help to reduce rough and flaky skin. They are frequently occlusive agents, i.e. substances that provide a layer of protection that help prevent moisture (water) loss from the skin. Examples for emollients are silicones, such as dimethicone or cyclomethicone, vegetable oils, butters such as coca butter or shea butter, alcohols such as stearyl alcohol or cetyl alcohol, and petrolatum derivatives such a petroleum jelly or mineral oil. Due to their electrical characteristics, i.e. resistivity and permittivity, most of the emollients show a poor electrostatic sprayability.

Moreover, even though some of the tanning formulations have appropriate electrical characteristics, it is still not possible to electrostatically spraying these formulations such that all parts of the skin are covered by the formulation. This result also in a streaking effect on the skin surface, i.e. the applied topical formulation creates a "zebra crossing" pattern on the skin.

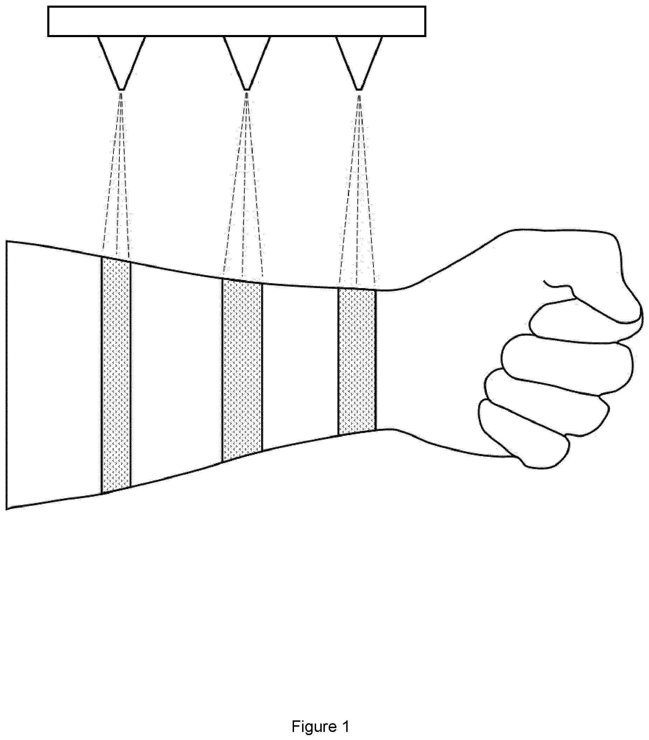

Figure 1 schematically shows the undesired "zebra crossing" pattern on the skin, which occurs if the topical cosmetic formulation is not uniformly sprayable and thus, uneven distribution of the formulation on the skin is possible.

Additionally, with respect to tanning formulations it has to be considered that most of the tanning formulations contain water in order to dissolve the water-soluble tanning agent(s) in the formulation. However, as for example described in US 2005/0100516, when water is atomized in an aerosol spray, the resulting particles have a tendency to be wet and course. This could cause the formulation to bead or clump on the skin and thus also resulting in an uneven or blotchy appearance.

Furthermore, another problem, which occurs with respect to tanning formulations, is that many tanning agents are unstable, in particular during storage, due to for example the reaction of the tanning agent with atmospheric oxygen.

Hence, object of the application was to provide an electrostatically sprayable topical formulation for giving skin a tanned appearance in the absence of ultraviolet radiation exposure or achieving a faster, darker tanned appearance in the presence of ultraviolet radiation exposure, which can be easily applied on the skin surface of a subject in a uniform manner in order to obtain a nearly full coverage of the skin surface and thus an even tanned appearance. Additionally, the topical (tanning) formulation should show a sufficient stability, in particular a sufficient storage stability.

It has been surprisingly found that in case the topical formulation comprises in addition to a tanning agent polar emollients and ethanol as well as water in certain amounts the topical formulation shows an improved electrostatic sprayability, i.e. in particular, no streaking effect occurs by applying the formulation on the skin surface of a subject. Hence, by electrostatically spraying of such a topical formulation a uniform and a nearly full coverage of the skin can be achieved. The use of a propellant for spraying the topical formulation of the invention on the skin surface of a subject is not necessary. Furthermore, it has been found that by using the topical formulation according to the invention, the uniform tanning appearance gets not lost while the tanning effect gradually subsides, because the topical formulation removes from the skin evenly.

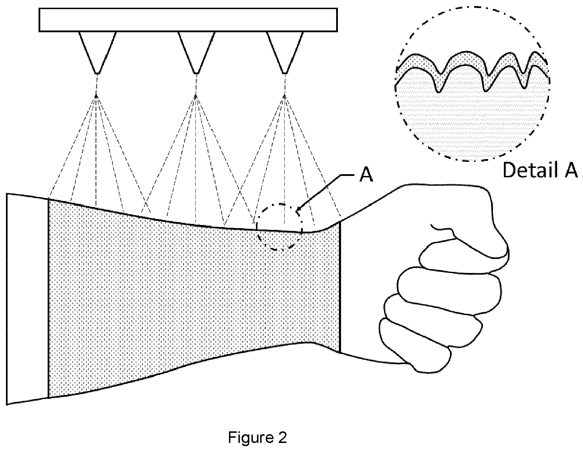

Figure 2 schematically shows the full coverage of the skin by using a topical formulation according to the invention, i.e. no streaking effect occurs. The enlarged section "Detail A" of Figure 2 shows a part of the skin, which is covered with the topical formulation of the invention.

Additionally, a sufficient stability, in particular a storage stability, without influencing the electrostatic sprayability of the formulation can be achieved in case the formulation contains an antioxidant.

Therefore, the present invention provides an electrostatically sprayable topical formulation, comprising

> a) 50 to 80 wt.-% ethanol;
> b) 2 to 15 wt.-% water;
> c) 5 to 25 wt.-% of at least one emollient, which is liquid at 20 °C and have a log $K_{ow}$ of 2 to 10; and
> d) 0.15 to 1 wt.-% of at least one antioxidant, based on the total weight of the formulation, and
> e) at least one tanning agent in an effective amount, wherein the sum of the weight of all components present in the topical formulation is 100 %.

The abbreviation "wt.-%" or "w/w", as used herein, means "weight percentage" and refers to the weight amount of a compound in relation to the (total) weight of a formulation of compounds or of a substrate if nothing else is explicitly stated or obvious under the circumstances.

The term "comprising" includes "consisting essentially of' and also "consisting of".

In the present specification, the use of "a" in the singular also comprises the plural ("some"), and vice versa, unless the context clearly indicates the contrary.

The topical formulation of the invention can be used alone or in a mixture comprising other cosmetic formulations. Preferably, the topical formulation is a tanning formulation.

The electrostatically sprayable topical formulation comprising the specific combination of components according to the invention preferably shows an specific electrical resistance of 15 to 100 GOhm·mm$^2$/m, more preferably of 20 to 85 GOhm·mm$^2$/m, it is even more preferred that the specific electrical resistance is not higher than 80, not higher than 60, not higher than 50 GOhm·mm$^2$/m, most preferably the electrostatic resistance is between 35 to 48 GOhm·mm$^2$/m, determined as described below (see measurement methods). This ensures that the formulation is good electrostatically sprayable and thus no undesired streaking effect on the skin occurs.

In addition, in order to provide a topical formulation which shows a good electrostatic spraying behavior, the formulation preferably has a surface tension of 15 to 50 mN/m, more preferably of 20 to 45 mN/m, 22 to 40 mN/m, more preferably 25 to 35 mN/m determined by the stamp method as described below (see measurement methods).

Furthermore, the relative permittivity of the topical formulation according to the invention may be at least 10.0, preferably at least 15.0, more preferably at least 20.0, most preferably at least 25.0. In particular, the relative permittivity of the topical formulation preferably is between 15 and 35.0, more preferably between 20 and 30.0, or between 22.0 and 28.0, most preferably between 25.0 and 27.0. The measurement method for determining the relative permittivity according to the invention is described below (see measurement methods).

Moreover, the topical formulation of the invention may have a viscosity of from 0.65 mPa·s to 5000 mPa·s, preferably of from 1.0 to 1000 mPa·s, 5.0 to 500 mPa·s, more preferably of from 10.0 to 100 mPa*s, determined as described below (see measurement methods). In particular a good spraying behavior of the topical formulation can be observed if the topical formulation has a viscosity below 4.0 mPa·s.

For spaying the topical formulation on the skin surface of a subject, every electrostatic device known in the art can be used. However, it is preferred to use a device as described in DE 10 2017 108 610.2, which is further specified in DE 10 2017 108 612.9, DE 10 2017 108 613.7, DE 10 2107 108 614.5 and DE 10 2107 108 615.3.

As mentioned above, the electrostatic sprayability of emollients used in topical cosmetic formulations is poor, in particular in case the emollients are non-polar emollients. Therefore, according to the invention the topical formulation comprises at least one polar emollient that is liquid at 20 °C.

In the cosmetic field, one possibility to define the polarity of a compound is to determine its n-octanol/water partition coefficient ($K_{ow}$ or P). The partition-coefficient refers to the ratio of concentrations of the compounds in the mixture of these two immiscible phases at equilibrium. Hence, the partition coefficient measures how hydrophilic ("water-loving") or hydrophobic ("water-fearing") the tested compound is. In most of the cases the n-octanol/water partition coefficient is stated as decade logarithms log $K_{ow}$ or log P. The determination of the n-octanol/water partition coefficient is described for example in J. Sangster, "Octanol-Water Partition Coefficients: Fundamentals and Physical Chemistry", Vol. 2 of Wiley Series in solution chemistry, John Wiley & Sons, Chichester, 1997.

According to the invention, the log $K_{ow}$ of the emollients, which are suitable for the topical formulation of the invention, is between 2.0 to 10.0, preferably between 2.5 and 9.5, 3.0 and 8.5, or between 3.5 and 7.5.

However, in most of the cases also the electrostatic spraying quality of polar emollients is not sufficient. It is known that the electrostatic spraying quality raises with the ethanol content. For example, the emollient octyldodecanol having a $K_{ow}$ of approx. 9.2 shows a poor electrostatic sprayability in particular at low voltages. However, in case octyldodecanol is mixed with ethanol, the spraying quality can be improved (see results of Table 1 below). The spraying test was carried out such that the distance from a spraying nozzles to the outer left side of a metal tube was 13 cm. The pre-set voltage of three subsequent tests were 20, 30 and 40 kV, respectively. For the fluid pump a voltage of 3.7 V was used. After changing each test liquid, the tube system was rinsed with isopropanol and/or ethanol. The spraying behavior of the formulations was optically evaluated and the spraying pattern was assessed to the following scale: good; moderate, poor and nearly not sprayable.

Table 1

| Formulation | **Electrical** conductivity [μm/cm] (at °C) | **Surface** tension [mN/m] (°C) (bubble pressure method; at 20s bubble lifetime) | Spraying results | | |
|---|---|---|---|---|---|
| | | | *Pre-set 20 kV* | *Pre-set 30 kV* | *Pre-set 40 kV* |
| Octyldodecanol (100 %) | 0.0 (24.0 °C) | 30.41 (22.8 °C) | no spraying at 16 kV | no spraying at 24 kV | nearly no spraying at 32 kV |
| Octyldodecanol (80 %) / Ethanol (20%) | 0.1 (24.0 °C) | 24.31 (22.3 C) | nearly no spraying at 16 kV | moderate spraying at 24 kV | good spraying at 34 kV |
| Octyldodecanol (60 %) / Ethanol (40 %) | 0.1 (24.5 °C) | 25.44 (22.3 °C) | nearly no spraying at 16 kV | moderate to good spraying at 24 kV | good spraying at 33 kV |

On the other hand, with respect to tanning formulations it has to be considered that most of the tanning agents are water soluble, for example dihydroxyacetone (DHA). Therefore, tanning formulations and thus the topical formulation of the invention have to contain a sufficient amount of water to ensure that the tanning agent is dissolved in the formulation. However, it has been found out that if the water content of the topical formulation is too high the electrostatic sprayability of the formulation gets worse. This is for example shown in Table 2 below. The spraying test used in the experiments of Table 2 was performed in the same manner as described with respect to the spraying test regarding the sprayability of octyldodecanol (see Table 1).

Table 2

| Formulation | Electrical conductivity [μm/cm] (at °C) | Surface tension [mN/m] (°C) (bubble pressure method; at 20s bubble lifetime) | Spraying results | | |
|---|---|---|---|---|---|
| | | | *Pre-set 20 kV* | *Pre-set 30 kV* | *Pre-set 40 kV* |
| Ethanol (90 %) / Water (10 %) | 0.4 (25.6 °C) | 22.96 (24.5 °C) | nearly no spraying at 16 kV | Poor to moderate spraying at 24 kV | good spraying at 33 kV |
| Ethanol (80 %) / Water (20%) | 0.4 (23.0 °C) | 23.93 (24.2 °C) | nearly no spraying at 16 kV | poor to moderate spraying at 24 kV | moderate to good spraying at 33 kV |
| Ethanol (60 %) / Water (40%) | 0.5 (at 23.1 °C) | 25.74 (24.2 °C). | nearly no spraying at 16 kV | poor spraying at 24 kV | moderate spraying at 33 kV |
| Ethanol (50 %) / Water (50%) | 0.6 (22.7 °C) | 26.58 (24.4 °C) | nearly no spraying at 16 kV | poor spraying at 24 kV | moderate spraying at 32 kV |

Hence, in order to ensure that that the topical formulation shows a sufficient spraying quality the amounts of ethanol and water present in the topical formulation of invention have to be in certain ranges.

According to the invention, in case the ethanol content is between 50 to 80 wt.-%, based on the total weight of the topical formulation, the electrostatic spraying behavior of the topical formulation shows the desired quality. In particular, it is preferred that ethanol content of the topical formulation is at least 52 wt.-%, at least 55 wt.-%, more preferably at least 58 wt.-%, but preferably not higher than 75 wt.-%, not higher than 70 wt.-%, more preferably not higher than 65 wt.-%, based on the total weight of the formulation. In a preferred embodiment of the invention, the ethanol content is between 53 and 62 wt.-%, based on the total weight of the formulation. In another preferred embodiment, the ethanol content is between 55 and 59 wt.-%, based on the total weight of the formulation.

In addition to the specific ethanol content, it is necessary that the water content of the topical formulation is between 2.0 to 15.0 wt.-%, preferably between 4.0 and 12.0 wt.-%, 5.0 and 11.0 wt.-% and more preferably between 6.0 and 10.5 wt.-%, based on the total weight of the formulation.

Moreover, since the topical formulation should show a sufficient skin caring effect, the topical formulation contains at least one emollient, preferably a mixture of emollients. In order to ensure that the good or even improved electrostatic spraying quality of the topical formulation is maintained the emollient(s) used in the topical formulation of the invention is liquid at 20 °C and polar. Examples which fulfills these requirements are known in the art. In particular, the emollients used in the topical formulation of the invention are preferably selected from the group consisting of octyldodecanol, dibutyl adipate, PPG-15 stearyl ether, PPG-3 myristyl ether, PPG-14 butyl ether, dicaprylyl carbonate, isoamyl cocoate, diethylhexyl carbonate, isopropyl myristate, isopropyl palmitate, decyl cocoate, phenoxyethyl caprylate, C12-C15 alkyl benzoate and combinations thereof. More preferably the emollients are selected from the group consisting of octyldodecanol, dibutyl adipate, PPG-15 stearyl ether, PPG-3 myristyl ether, PPG-14 butyl ether and mixtures thereof.

It is preferred that the topical formulation of the invention comprises at least two or more, at least three, but not more than five different emollients. In particular, it is preferred that the topical formulation comprises a mixture of octyldodecanol and dibutyl adipate.

The different emollients are used in different or equal amounts in the topical formulation of the invention so that the total amount of emollients is between 5 to 25 wt.-%, based on the total weight of the formulation. Preferably, the total amount of emollients present in the formulation is from 6 to 20 wt.-%, 8 to 15 wt.-%, more preferably from 10 to 12 wt.-%, based on the total weight of the formulation.

The invention relates to a topical (tanning) formulation that provides a tanned appearance in the absence of ultraviolet radiation exposure or a faster, darker tanned appearance in the presence of ultraviolet radiation exposure. Therefore, the topical formulation of the invention comprises at least one tanning agent in an effective amount.

Self-tanning agents are generally selected from among mono- or polycarbonyl compounds. Suitable tanning agents for the topical formulation of the invention are isatin, alloxan, ninhydrin, glyeraldehydes, mesotartaric, aldehyde, glutaraldehyde, erythrulose, dihydroxyacetone (DHA), derivatives thereof, and combinations thereof. In particular it is preferred that the tanning agent is dihydroxyacetone (DHA), erythrulose or a mixture thereof. As for example disclosed in US 6 451 293 the combination of erythrulose with a reducing sugar, such as DHA, provides an even longer lasting tanned appearance. In case the reducing sugar is used in combination with erythrulose it preferred that the ratio of reducing sugar to erythrulose is 1:10 to 10:1, preferably 1:1, 3:1 or 2:1.

DHA is a 3-carbon sugar that when applied to the skin causes a chemical reaction with amino acids in the surface cells of the skin producing darkening effect. Other reducing sugars such as glucose, xylose, fructose, reose, ribose, arabinose, allose, tallose, altrose, mannose, galactose, sucrose, and lactose may be also suitable for the formulation of the invention.

An effective amount of tanning agent in the meaning of the invention is an amount, which results in a color change (tanning effect) of the treated skin noticeable by a subject. The color change can be also determined by measuring the increase of melanin deposition in pigmented epidermal equivalents as described for example in EP 2 191 819, or by using the in vivo skin darkening test measuring the change in lightness as described for example in EP 2 198 848. In particular, an amount of at least 0.5 wt.-% of the tanning agent, based on the total weight of the topical formulation, is an effect amount in the meaning of the invention. Preferably, the amount of the tanning agent present in the topical formulation is between 0.5 and 10.0 wt.-%, between 1.0 and 8.0 wt.-%, more preferably between 2.0 and 6.0 wt.-%, based on the total weight of the formulation.

One of the main problems which occurs with self-tanning agents is their instability, in particular during storage. In case DHA is used as tanning agent it is known that during storage the formulation release formaldehyde, which is harmful to health and produces a bad odor. For commercial use it is desirable that the tanning formulation of the invention is stable for at least 6 months.

In order to ensure that the tanning agent is stable during storage, the topical formulation of the invention contains at least one antioxidant in an amount of 0.15 to 1.0 wt.-%, based on the total weight of the formulation. This amount ensures the stability of the tanning agent without negatively influencing the good electrostatic spraying quality of the formulation. It is preferred that the amount of the at least one antioxidant is between 0.18 and 0.8 wt.-%, more preferably between 0.2 and 0.6 wt.-% or 0.3 and 0.5 wt.-% based on the total weight of the formulation.

According to Römpp Chemie Lexikon, Stuttart/NewYork, Georg Thieme Verlag, 1995, antioxidants are compounds that inhibit or prevent undesired change in the substances to be protected caused by the action of oxygen, e.g. oxidative processes. Antioxidants are known in the prior art and for example described in Kosmetik International 2013 (8), pages 12-15. Examples for antioxidants suitable for cosmetic topical formulations are phenols, hydroquinones, pyrocatechols, sulphurous acid and salts thereof, aromatic compounds and amines, each of which may be substituted by sterically hindering groups, and metal complexes thereof.

Preferably, the topical formulation of the invention comprises at least one antioxidant selected from the group consisting of anoxomer, ascorbyl palmitate, butylated hydroxyanisole, butylated hydroxytolune, hypophosphorous acid, potassium metabisulfite, propyl octyl and dodecyl gallate, sodium metabilsulfite, sodium dioxide, tocopherols bis-ethyhexyl-hydroxydiemethodxy-benzylmalone and mixtures thereof.

It is more preferred that the antioxidant used in the formulation of the invention is bis-ethyhexyl-hydroxydiemethodxy-benzylmalone. In that case it is preferred that the amount of antioxidant present in the formulation is between 0.15 and 0.6 wt.-%, preferably, between 0.15 and 0.5 wt.-%, more preferably between 0.15 and 0.3 wt.-%, based on the total weight of the formulation.

The topical formulation of the invention shows a stability of at least 6 months, more preferably of at least 12 months. In particular, the topical formulation shows a stability of 6 to 18 months, or preferably of 8 to 12 months.

In order to further improve the skin caring effect of the formulation, it is further preferred that the topical formulation comprises in addition to the polar emollients at least one moisture agent. The moisture agent(s) present in the topical formulation differs from the polar emollients. The amount of the moisture agent present in the formulation is preferably at least 2.0 wt.-%, at least 3.0 wt.-%, at least 4.0 wt.-% and, preferably, not higher than 12.0 wt.-%, not higher than 11.0 wt.-%, more preferably not higher than 10.5 wt.-%, based on the total weight of the formulation.

Furthermore, it is preferred that the topical formulation comprises a mixture of different moisture agents, preferably a mixture of at least two, at least three, at least four or more different moisture agents, but preferably not more than eight, not more than six different moisture agents.

Specific examples for moisture agents known in the art and suitable for the invention are fructose, glucose, maltitol, maltose, mannitol, glycerin, glycerol polymers, propylene glycol, 1,3 butylene glycol, inositol, lactitol, linolenic acid, but also honey, beeswax, hyaluronic acid, hydrogenated starch hydrolysate, natural moisturizing factor, acetylated lanolin and alcohol thereof, alanine, aspartic acid, barrier sphingolipids, ceramides, ceresin, collagen, collagen, amino acids, serum protein, or extracts and oils of plants, like canola oil, algae extract, aloe barbadensis, aloe-barbadensis extract, aloe barbadensis gel, avocado (*persea gratissima*) oil, Calendula officinalis extract and oil thereof, hydrogenated palm kernel oil, jojoba *(buxus chinensis)* oil, althea officinalis extract, apricot (*prunus armeniaca*) kernel oil, arnica montana extract, birch (*betula alba*) bark extract, borage (*Borago officinalis*) extract, butcherbroom (*ruscus aculeatus*) extract, candelilla (*euphorbia cerifera*) wax, canola oil, cardamon (*elettaria cardamomum*) oil, carnauba (*copernicia cerifera*) wax, carrot (*Daucus carota sativa*) oil, castor (*Ricinus communis*) oil, chamomile (*anthemis nobilis*) oil, clary (*salvia sclarea*) oil, cocoa (*Theobroma* cacao) butter, coconut (*Cocos nucifera*) oil, corn (Zea *mays)* oil, eucalyptus globulus oil, evening primrose (*Oenothera biennis*) oil, nut oils for example macadamia ternifolia nut oil, olive (*Olea*

*europaea*) oil lavender (*Lavandula angustifolia*) oil, lemon (*citrus medica limonum*) oil, rice (*Oryza sativa*) bran oil, peppermint (*Mentha piperita*) oil, rosemary (*Rosmarinus officinalis*) oil, rose oil, safflower (*carthamus tinctorius*) oil, sage (*salvia officinalis*) oil, sandalwood (*santalum album*) oil, sesame (*Sesamum indicum*) oil, silk powder, sunflower (*Helianthus annuus*) seed oil sweet almond (*prunus amygdalus dulcis*) oil, soybean (*glycine soja*) oil, wheat (*Triticum vulgare*) germ oil, and ylang ylang (*cananga odorata*) oil, etc.

In particular it is preferred that the topical formulation of the invention comprises at least one moisture agent selected from the group consisting of fructose, glucose, glycerin, propylene glycol, 1,3 butylene glycol, linolenic acid, hyaluronic acid, beeswax, hydrogenated starch hydrolysate, acetylate lanolin alcohol, ceramides, ceresin, collagen, collagen, algae extract, sweet almond oil, aloe barbadensis, aloe-barbadensis extract, aloe barbadensis gel, avocado oil, canola oil, *Calendula officinalis* extract, hydrogenated palm kernel oil, jojoba oil and mixtures thereof.

More preferably, the moisture agent is selected from the group consisting of glycerin, propylene glycol, aloe barbadensis, aloe-barbadensis extract, aloe barbadensis gel, jojoba oil and mixtures thereof. In a further preferred embodiment of the invention the moisture agent is at least glycerin, propylene glycol and/or aloe-barbadensis extract.

In common, topical cosmetic formulations comprise in addition to the above described active compounds also cosmetic additives such as cosmetically acceptable carriers, oils, sterols, amino acids powders, colorants, pigments, dyes, pH adjusters, perfumes, essential oils, vitamins E, pro-vitamins, essential fatty acid, sphingolipids, UV filter etc. and combinations thereof. These additives and combinations thereof may be also present in the topical formulation of the invention.

Preferably, according to the invention, the additives are selected from the group consisting of perfumes, organic acids such as citric acid, vitamins and/or pro-vitamins such as tocopherol and/or tocopheryl acetate, and combinations thereof. It is particular it is preferred that the formulation includes at least tocopherol, in particular alpha-tocopherol, as additive. Tocopherols are a class of organic compounds, many of which have vitamin E activity and are often used in cosmetic formulations. Tocopherol has antioxidant properties that increase the moisture content of the skin, improve the skin surface and slow down aging processes by smoothing minor wrinkles.

Furthermore, it is preferred that the formulation comprises citric acid for pH adjustment of the formulation. In particular, it is preferred that an aqueous solution of citric acid is used for the pH adjustment, more preferably a 20% aqueous solution of citric acid is used.

The topical formulation of the invention comprises 0 to 4 wt.-% of at least one cosmetic additive, preferably 0.01 to 3.0 wt.-%, 0.1 to 2.5 wt.-%, more preferably 0.2 to 2 wt.-% of at least one cosmetic additive, based on the total weight of the formulation. If the topical formulation comprises at least alpha-tocopherol as additive it is further preferred that the amount of alpha-tocopherol is from 0.01 to 0.3 wt.-%, preferably from 0.05 to 0.25 wt.-%, or 0.08 to 0.22 wt.-%, more preferably from 0.1 to 0.2 wt.-%, based on the total weight of the formulation.

Furthermore, due to the good electrostatic sprayability of the topical formulation of the invention, there is no need that the formulation further comprises a propellant in order to ensure that the formulation is sprayable.

In cosmetic and dermatology industry different propellants are used, for example, chemicallyinert hydrocarbons such as propane, n-butane, isobutene and cyclopropane, and mixtures thereof, as well as halogenated hydrocarbons such as for example dichlorodifluormethane or 1,1-dichloro-1,1,2,2-tetrafluoroethane. isobutane, used singly or mixed with other hydrocarbons, particularly propane is for example preferred used in aerosol. The use of propellants involves numerous disadvantages that can be avoided by providing the formulation of the invention.

In a further embodiment of the invention the electrostatically sprayable cosmetic formulation comprises

a) 50 to 65 wt.-% ethanol;
b) 5 to 11.5 wt.-% water;
c) 8 to 20 wt.-% of at least one emollient, which is liquid at 20 °C and have a log $K_{ow}$ of 2 to 10;
d) 0.15 to 0.6 wt.-% of at least one antioxidant,
e) 1 to 8 wt.-% of at least one tanning agent
f) 2 to 10.5 wt.-% of at least one moisture agent;
g) 0.15 to 2.5 wt.-% of at least one cosmetic additive, based on the total weight of the formulation and wherein the sum of the weight of all components present in the topical formulation is 100 %.

In particular, it is preferred that the electrostatically sprayable cosmetic formulation consists of

a) 50 to 61.5 wt.-% ethanol;
b) 8 to 11 wt.-% water;
c) 8 to 20 wt.-% of a mixture of emollients consisting of dibutyl adipate and octyldodecanol;
d) 0.15 to 0.5 wt.-% bis-ethylhexyl hydroxymethoxybenzlymalonate as antioxidant;
e) 2 to 8 wt.-% dihydroxyacetone as tanning agent;
f) 2 to 10.5 wt.-% of a mixture of moisture agents consisting of propylene glycol, glycerin and aloe barbadensis extract;

g) 0.15 to 2.5 wt.-% of a mixture of cosmetic additives consisting of alpha-tocopherol, tocopheryl acetate, citric acid and perfume, based on the total weight of the formulation and wherein the sum of the weight of all components present in the topical formulation is 100 %.

More preferred is a electrostatically sprayable cosmetic formulation consists of

a) 50 to 61 wt.-% ethanol;
b) 5 to 11 wt.-% water;
c) 8 to 18 wt.-% of a mixture of emollients consisting of 6 to 15 wt.-% dibutyl adipate and 2 to 12 wt.-% octyldodecanol; and
d) 0.15 to 0.5 wt.-% bis-ethylhexyl hydroxymethoxybenzlymalonate,
e) 2 to 8 wt.-% dihydroxyacetone
f) 2.5 to 10.5 wt.-% of a mixture of moisture agents consisting of 1 to 8 wt.-% propylene glycol, 1 to 6 wt.-% glycerin and 0.5 to 2 wt.-% aloe barbadensis extract;
g) 0.17 to 2.0 wt.-% of a mixture of cosmetic additives consisting of 0.05 to 0.15 wt.-% alpha-tocopherol, 0.1 to 0.3 wt.-% tocopheryl acetate, 0.01 to 0.02 wt.-% citric acid, 0.01 to 0.1 wt.-% perfume, based on the total weight of the formulation and wherein the sum of the weight of all components present in the topical formulation is 100 %.

The examples that follow are intended for illustrating the invention in more detail.

## Examples

## Measurement methods

## 1. Specific electrical resistance

The specific electrical resistance was determined by the means of an Electrospray Paint Test Meter of DCA Electronic Ltd. The measuring current was between 85 $\mu$A and 100 $\mu$A or higher. The measuring range was 10 kOhm up to 5 MOhm. The measured electrical resistance was approximately conversed to the specific electrical resistance according to ASTDM D5682-08.

## 2. Surface tension

The surface tensions of the formulations as described herein were measured by two different measurement methods, the stamp method and the bubble pressure method. Due to the low viscosity of the cosmetic formulations according to the invention, the stamp method was used to determine the surface tension of these cosmetic formulations.

### 2.1 Stamp method

The metal stamp can be used to determine the surface tension of liquids and highly viscous media (e.g. polymer melt or adhesives). The liquid is applied to the stamp, which forms an entire surface and a drop on the stamp. The stamp material has no influence on the measurement result.

The surface tension of the liquid can be calculated from the drop contour and the density of the liquid using the SCA 20 software (www.dataphysics-instruments.com, DATAPhysics OCA20) and the Young-Laplace equation.

In addition to the surface tension, the contact angle and the volume of the drop are also measured. The determined surface tension is independent of the contact angle. The same surface tension values are measured both when the droplet is enlarged and when it is reduced by suction.

*2.2 Bubble pressure method*

With the bubble pressure method the dynamic surface tension of the formulations in liquids can be determined. This method is carried out by using the tensiometer SITA pro line t15 (www.sita-messtechnik.de), wherein an air stream is lead into the sample liquid through a robust capillary and the pressure development necessary for the bubble generation is measured at room temperature (automatic mode: bubble lifetime from 0.02 to 20 s. It is preferred to indicate the surface tension s [mN/m] at a bubble lifetime of 20 s.

3. Relative permittivity

The relative permittivity was determined by the means of a High Frequency Liquid Dielectric Constant Meter (Time Domain Reflection) "ALPHA TDR-5000" of Zadow Electronics (ww.zadow-electronics.de) having two parallel wire sensors that have a length of 5 cm. The measurement method was carried out such that the TDR-500 periodically sends voltage pulses with times (250 ps) onto the sensor cable to determine the dielectric constant (K) of the formulations:

$$K = 15^2 \times (\frac{t}{r})^2$$

t = running time in ns; the running time was 5 ns
r = length of the wire sensor (5 cm)

The relative permittivity of the formulation was calculated on the basis of the determined dielectric constant.

4. Electrical conductivity

The electrical conductivity was measured by means of Sartorious PT-20, hand-held conductivity meter, measuring cell PY-CL1 (www.sartorius.de). Measurement at room temperature.

5. Viscosity

The viscosity was determined by means of Paar Physica UDS 200 coaxial cylinder Rheometer. The measurement was performed at 22 °C and with an increasing shear rate of 1 up to 3000 s$^{-1}$.

6. Storage Stability

The tested formulations were stored for four weeks at 40 °C. These conditions correspond to usual storage conditions of a cosmetic formulation for one year.

The storage stability of the formulation is defined by the amount of released formaldehyde after storage of the sample (T1). The smaller the amount of released formaldehyde, the more stable the formulation.

For determining the amount of released formaldehyde after storage (T1), the amount of free formaldehyde present in the sample before storage (T0) and after storage (T2) was measured by the following method: The samples were weighed into a screw cap bottle and the free formaldehyde was then extracted with a solution of dichlormethan and 0.002M HCl for 20 minutes. For better phase separation, the sample was centrifuged. Afterwards, the aqueous phase was filtered, bottled and the amount of free formaldehyde was directly measured by the means of HPLC 3 and SOP M3130 in a HPLC/UV and fluorescence, post-column derivatization method.

The amount of released formaldehyde after storage (T1) was calculated according to the following formulation: T1 = T2 - T0.

7. Spraying test

The conditions for all test setups were the same. The tested cosmetic formulations were sprayed by using an IONIQ spraying system, which is described in detail in DE 10 2017 108 610.2, DE 10 2017 108 612.9, DE 10 2017 108 613.7, DE 10 2107 108 614.5 and.DE 10 2107 108 615.3. After spraying the IONIQ system was cleaned with denaturized alcohol. The voltage of the pump for spraying was 3.6 V.

In order to determine whether the topical formulation provides a homogenous distribution and a sufficient tanned appearance on the skin of a subject, the topical formulation was applied on the skin of the subject in the following manner: The tested cosmetic formulation was slowly sprayed on the whole body of the test person. The spraying time was approx. 15 second per arm and 30 second per leg. The formulation was leak to soak for 10 minutes. After 8 hours the subject take a shower and assesses the spraying result.

**Example 1**

Compositions of the formulations:

Table 3

| Material | INCI | Formulation 1 [%] | Formulation 2 [%] |
|---|---|---|---|
| Dihydroxyacetone (DHA), extra pure | DIHYDROXYAETONE | 5 | 5 |
| Water, demin. | AQUA | 10 | 10 |
| Ethanol, 96% | ALCOHOL DENAT. | 50 | 59.8 |
| Tegosoft E (emollient) | PPG-15 STEARYL ETHER | 30 | 20 |
| Glycerin Ph. Eur 85% (moisture agent) | GLYCERIN | 5 | 5 |
| Perfume | PERFUME | - | 0.2 |

The spraying properties of both formulations were tested by using spraying test as described above.

Even though the amount of ethanol and water of Formulation 1 was in accordance with the teaching of the invention, due to the high amount of emollient present in the formulation, the spraying behavior of Formulation 1 does not show the desired quality, i.e. homogenous distribution on the skin of the subject, in comparison to Formulation 2. Example 1 demonstrates that only a formulation, which comprises the specific combination of compounds according to the invention, provides the desired spraying properties.

**Example 2**

Compositions of the formulations:

Table 4

| Material | INCI | Formulation 3 [%] | Formulation 4 [%] | Formulation 5 [%] |
|---|---|---|---|---|
| **Dihydroxyaceton (DHA),** extra pure | DIHYDROXYACETONE | 5 | 5 | 5 |
| **Water,** demin. | AQUA | 10 | 10 | 10 |
| **Ethanol** (98%) | ALCOHOL DENAT. | 58.95 | 58.45 | 58.75 |
| Cetiol B | DIBUTYL ADIPATE | 12 | 12 | 12 |
| Eutanol G | OCTYLDODECANOL | 3 | 3 | 3 |
| **Total amount of emollients** | | 15 | 15 | 15 |
| Propylenglycol Ph. Eur. 7.0 | PROPYLENE GLYCOL | 5 | 5 | 5 |
| Glycerin Ph. Eur. 85% | GLYCERIN, AQUA | 4 | 4 | 4 |
| Aloe (baradensis) Herbasol Extract IPM | ALOE BARBADENSIS LEAF EXTRACT, ISOPROPYL MYRISTATE | 1 | 1 | 1 |
| **Total amount of moisture agents** | | 10 | 10 | 10 |
| **Antioxidant: RonaCare AP™** | BIS-ETHYHEXYL HYDROXYMETHOXY BENZYLMALONATE | - | 0.5 | 0.2 |
| DL-alpha-Tocopherol | TOCOPHEROL | 0.1 | 0.1 | 0.1 |
| DL-alpha-Tocopherolacetat | TOCOPHERYL ACETATE | 0.2 | 0.2 | 0.2 |
| Citric acid (20% aq.) | AQUA, CITRIC ACID | 0.05 | 0.05 | 0.05 |
| Perfume | PERFUME | 0.7 | 0.7 | 0.7 |
| **Total amount of additives** | | 1.25 | 1.25 | 1.25 |

All formulations of Table 4 showed the desired spraying behavior. In particular, all formulations had a relative permittivity, specific electrical resistance, surface tension and viscosity in accordance with the invention (see e.g. Formulation 3).

Table 5

| Property | Formulation 3 |
|---|---|
| Relative permittivity | 26.32 |
| Specific electrical resistance [GOhm·mm$^2$/m] | 21.1 |
| Surface tension (stamp method) [mN/m] | 25.81 (density: 0.892 [g/cm$^3$]) |
| Viscosity [mPa*s] | 2.9 |

However, only in case the formulation contains additionally to tocopherol an antioxidant, the desired storage stability was obtained. Furthermore, it was found out that if the amount of antioxidant used in the formulation is too high, the stability gets worse.

Table 6

| Formulation | Antioxidants | Concentration of released Formaldehyde (T1) [%] |
|---|---|---|
| Formulation 3 | 0.1% Tocopherol | 0.017 |
| Formulation 4 | 0.1% Tocopherol + 0.5% RonaCare AP | 0.0075 |
| Formulation 5 | 0.1% Tocopherol + 0.2% RonaCare AP | < 0.0005 |

The concentration of released formaldehyde of Formulation 5 of Table 4 corresponds a storage stability of at least 12 months. Hence, the Formulation 5 of Table 4 provides all desired properties, i.e. good spraying behavior, homogenous distribution on the skin and storage stability, together.

**Example 3**

Formulation 5 of the above Table 4 was tested by 36 consumers to demonstrate the benefits of the topical formulation according to the invention. The formulation was applied on the skin by each consumer her/himself according to the Spraying test as described above. Some of the consumers were already familiar with the spraying system some of them use the system firstly. The consumer rated the topical formulation and its application as followed:

*Ease of use/convenience*

The product was easy to apply - 88 % agreement
The skin feeling during application on the skin was pleasant - 92 % agreement
The skin feeling after application on the skin was pleasant - 94 % agreement

*Quality of tan*

The product provides a natural, healthy looking tan - 72 % agreement
The product provides the desired intensity of tan - 72 % agreement
The tan is even and streak free - 72 % agreement
The skin caring effect of the product is satisfactory or better - 89 % agreement
Furthermore, 82% of the consumers agreed that the uniform appearance gets not lost while the tanning effect subsides, because the product removes from the skin evenly after tanning the skin.

*Overall Rating*

86 % of the consumers agreed that the product provides the desired sprayability and tanning appearance.

**Claims**

1. Electrostatically sprayable topical formulation, comprising

   a) 50 to 80 wt.-% ethanol;
   b) 2 to 15 wt.-% water;
   c) 5 to 25 wt.-% of at least one emollient, which is liquid at 20 °C and have a log $K_{ow}$ of 2 to 10;
   d) 0.15 to 1 wt.-% of at least one antioxidant, based on the total weight of the formulation, and
   e) at least one tanning agent in an effective amount,

   wherein the sum of the weight of all components present in the topical formulation is 100 %.

2. Electrostatically sprayable topical formulation according to claim 1, wherein the effective amount of the tanning agent is between 0.5 and 10 wt.-%, based in the total weight of the formulation.

3. Electrostatically sprayable topical formulation according to claim 1 or 2, wherein the topical formulation further comprises at least one moisture agent in an amount of 2 to 12 wt.-%, based on the total weight of the formulation.

4. Electrostatically sprayable topical formulation according to any one of the previous claims, wherein the topical formulation further comprises at least one cosmetic additive in an amount of 0.01 to 4.0 wt.-%, based on the total weight of the formulation.

5. Electrostatically sprayable topical formulation according to any one of the previous claims, wherein the amount of at least one antioxidant is between 0.15 and 0.5 wt.-%, based on the total weight of the formulation.

6. Electrostatically sprayable topical formulation according to any one of the previous claims, wherein the tanning agent is dihydroxyacetone.

7. Electrostatically sprayable topical formulation according to any one of the previous claims, wherein the at least one emollient is selected from the group consisting of octyldodecanol, dibutyl adipate, PPG-15 stearyl ether, PPG-3 myristyl ether and PPG-14 butyl ether or combinations thereof.

8. Electrostatic sprayable topical formulation according to any one of the previous claims, wherein the antioxidant is bis-ethyhexyl-hydroxydimethoxy-benzylmalonate.

9. Electrostatically sprayable topical formulation according to any one of claims 3 to 8, wherein the formulation comprises a mixture of moisture agents.

10. Electrostatically sprayable topical formulation according to any one of claims 3 to 9, claims, wherein the moisture agent is selected from the group consisting of glycerin, propylene glycol, aloe barbadensis, aloe-barbadensis extract, aloe barbadensis gel, jojoba oil and mixtures thereof.

11. Electrostatically sprayable topical formulation according to any one of claims 4 to 10, wherein the at least one cosmetic additive is selected from the group consisting of organic acids, perfumes, vitamin, pro-vitamins and combinations thereof.

12. Electrostatically sprayable topical formulation according to any one of claims 4 to 11, wherein the formulation comprises at least tocopherol as cosmetic additive.

13. Electrostatically sprayable topical formulation according to any one of the previous claims, wherein the formulation does not comprise a propellant.

14. Electrostatic sprayable topical formulation according to any one of the previous claims, wherein the topical formulation comprises

    a) 50 to 70 wt.-% ethanol;
    b) 5 to 12 wt.-% water;
    c) 8 to 22 wt.-% of at least one emollient, which is liquid at 20 °C and have a log $K_{ow}$ of 2 to 10;
    d) 0.15 to 0.8 wt.-% of at least one antioxidant;
    e) 1 to 8 wt.-% of at least one tanning agent
    f) 2.5 to 11 wt.-% of at least one moisture agent, and
    g) 0.1 to 2.5 wt.-% of at least one cosmetic additive, based on the total weight of the formulation, wherein the sum of the weight of all components present in the topical formulation is 100 %.

15. Use of an electrostatic sprayable topical formulation according to any one of claims 1 to 14, wherein the topical formulation is topically applied to the skin of a subject by electrostatic spraying.

Figure 1

Figure 2

Europäisches
Patentamt

European
Patent Office

Office européen
des brevets

# EUROPEAN SEARCH REPORT

Application Number

EP 21 16 3633

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 00/54892 A1 (COOPER TROY HOWARD [US]) 21 September 2000 (2000-09-21) * page 11, line 30 - page 12, line 4; claim 31 * ----- | 1-15 | INV. A61K8/34 A61K8/35 A61K8/67 A61K8/45 A61Q19/04 A61K8/37 A61K8/49 |
| A | US 6 251 374 B1 (LAUGHLIN THOMAS J [US]) 26 June 2001 (2001-06-26) * column 1, lines 20-22 * * column 3, lines 14-32; examples 1-6 * * column 9, lines 58-61 * * column 8, lines 8-13 * ----- | 1-15 | |
| A | WO 2007/057686 A1 (POTTS TARIQ [GB]) 24 May 2007 (2007-05-24) * the whole document * ----- | 1-15 | |
| A | GB 2 273 872 A (UNILEVER PLC [GB]) 6 July 1994 (1994-07-06) * the whole document * ----- | 1-15 | |
| A | EP 0 523 961 A1 (UNILEVER PLC [GB]; UNILEVER NV [NL]) 20 January 1993 (1993-01-20) * the whole document * ----- | 1-15 | TECHNICAL FIELDS SEARCHED (IPC) A61K A61Q |
| A | EP 0 812 586 A1 (BOOTS CO PLC [GB]) 17 December 1997 (1997-12-17) * the whole document * ----- | 1-15 | |

The present search report has been drawn up for all claims

| Place of search | Date of completion of the search | Examiner |
|---|---|---|
| Munich | 19 October 2021 | Nopper, Agathe |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 16 3633

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2021

| Patent document cited in search report | | | Publication date | Patent family member(s) | | | Publication date |
|---|---|---|---|---|---|---|---|
| WO 0054892 | | A1 | 21-09-2000 | AT | 333952 | T | 15-08-2006 |
| | | | | AU | 777395 | B2 | 14-10-2004 |
| | | | | CA | 2367404 | A1 | 21-09-2000 |
| | | | | DE | 60029569 | T2 | 26-07-2007 |
| | | | | EP | 1161306 | A1 | 12-12-2001 |
| | | | | ES | 2269114 | T3 | 01-04-2007 |
| | | | | JP | 4538590 | B2 | 08-09-2010 |
| | | | | JP | 2002538910 | A | 19-11-2002 |
| | | | | MX | PA01009140 | A | 14-07-2003 |
| | | | | US | 6387081 | B1 | 14-05-2002 |
| | | | | WO | 0054892 | A1 | 21-09-2000 |
| US 6251374 | | B1 | 26-06-2001 | NONE | | | |
| WO 2007057686 | | A1 | 24-05-2007 | NONE | | | |
| GB 2273872 | | A | 06-07-1994 | NONE | | | |
| EP 0523961 | | A1 | 20-01-1993 | AU | 1967292 | A | 21-01-1993 |
| | | | | BR | 9202672 | A | 23-03-1993 |
| | | | | CA | 2073557 | A1 | 16-01-1993 |
| | | | | EP | 0523961 | A1 | 20-01-1993 |
| | | | | JP | H05194145 | A | 03-08-1993 |
| | | | | US | 5268166 | A | 07-12-1993 |
| | | | | ZA | 925271 | B | 17-01-1994 |
| EP 0812586 | | A1 | 17-12-1997 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2949403 A
- EP 0302147 A
- EP 2198847 A
- US 20050100516 A
- US 20030094510 A
- WO 9411119 A
- WO 2001012139 A
- US 20100116897 A

- DE 102017108610
- DE 102017108612
- DE 102017108613
- DE 102107108614
- DE 102107108615
- US 6451293 B
- EP 2191819 A
- EP 2198848 A

**Non-patent literature cited in the description**

- Octanol-Water Partition Coefficients: Fundamentals and Physical Chemistry. **J. SANGSTER.** Wiley Series in solution chemistry. John Wiley & Sons, 1997, vol. 2

- Römpp Chemie Lexikon. Georg Thieme Verlag, 1995
- *Kosmetik International,* 2013, vol. 8, 12-15